Europäisches Patentamt

(19) European Patent Office

Office européen des brevets

(11) Publication number: **0 099 202**
A1

(12) **EUROPEAN PATENT APPLICATION**

(21) Application number: **83303724.5**

(22) Date of filing: **28.06.83**

(51) Int. Cl.³: **G 01 N 33/22**
**G 01 N 1/20**

(30) Priority: **06.07.82 US 395427**

(43) Date of publication of application:
**25.01.84 Bulletin 84/4**

(84) Designated Contracting States:
**DE FR GB IT**

(71) Applicant: **THE BABCOCK & WILCOX COMPANY**
**1010 Common Street P.O. Box 60035**
**New Orleans Louisiana 70160(US)**

(72) Inventor: **Bohl, Thomas L.**
**306 Hyder Drive**
**Madison Ohio 44057(US)**

(74) Representative: **Cotter, Ivan John et al,**
**D. YOUNG & CO. 10 Staple Inn**
**London WC1V 7RD(GB)**

(54) Methods and apparatus for the on-line analysis of coal.

(57) An apparatus for the on-line analysis of coal comprises four radial arms (16) that extend from an output shaft (28) of an indexing motor (14). Sample cups (12) at the ends of the arms (16) are indexed along a circular path (13) past a filling station (18) where the cup is filled with pulverised coal, an analysing station (20) where various chemical analyses are performed on the coal sample, a dumping station (22) where the residue of the coal sample is dumped for disposal, and a cleaning station (24) where the sample cup is cleaned in preparation for another analysis cycle.

EP 0 099 202 A1

Croydon Printing Company Ltd

1

# METHODS AND APPARATUS FOR
## THE ON-LINE ANALYSIS OF COAL

This invention relates to methods and apparatus for the on-line analysis of coal.

Coal analysis in the facilities of large coal users is generally accomplished by traditional laboratory techniques performed on coal grab samples. Such sampling requires a plurality of manual manipulations, and the analyses are very time consuming.

Some attempts have recently been made to provide on-line, real time analyses. For example, automated instruments have been developed using radiation techniques wherein an instrument straddles a coal feeder belt and irradiates the coal with neutrons or gamma radiation. Re-radiation of the elements of the coal is detected and the coal constituents are determined by computer analysis.

While such devices are effective, they are very large and expensive, and since they deal with inherently dangerous materials they are subject to extensive government licensing and safety procedures which add to the total cost of operation.

Because of the foregoing, it has become desirable to develop an on-line, radiation-free, automatic system for analysing coal samples.

According to the invention there is provided a method for the on-line analysis of coal, the method being characterised by the steps of filling a sample cup of known volume with pulverised coal at a first station, indexing the sample cup to a second station, performing one or more analysis operations on the coal sample at the second station, indexing the sample cup to a third station, dumping the residue of the coal sample at the third station, indexing the sample cup to a fourth station, cleaning the sample cup at the fourth station, and returning the sample cup to the first station.

The invention also provides apparatus for the on-line analysis of coal, the apparatus being characterised by a sample cup of predetermined volume, a first station including means for introducing a sample of pulverised coal into the sample cup, a second station including means for performing one or

2

more analysis operations on the sample, a third station including means for receiving the residue of the sample after completion of the analysis operation or operations, a fourth station including means for cleaning the sample cup, and means for indexing the sample cup sequentially from the first through to the fourth stations and back to the first station.

A preferred embodiment of the present invention described hereinbelow solves or at least alleviates the aforementioned problems associated with the prior art by providing a method and apparatus for automatically weighing coal samples and performing chemical analyses of the samples. The apparatus can be very compact and relatively inexpensive to construct and maintain. Also, no inherently hazardous constituents are used in the analyses.

More specifically, the preferred embodiment of the invention includes a plurality of stations distributed about a central indexing motor drive. A plurality of sampling cups are attached to rotary arms attached to the motor drive. The analysing apparatus includes a sampling station where coal is automatically extracted from a main coal feed system, an analysing station where all the chemical analyses are performed, a dumping station where coal residue is dumped from the sampling cups, and a cleansing station where the sampling cups are cleaned in preparation for the next sampling cycle. There is a sampling cup for each station so that the various steps in the process are performed simultaneously as the sampling cups are indexed to each station. A strain gauge or similar device is provided on each sampling arm so that the weight of each sampling cup can be simultaneously monitored.

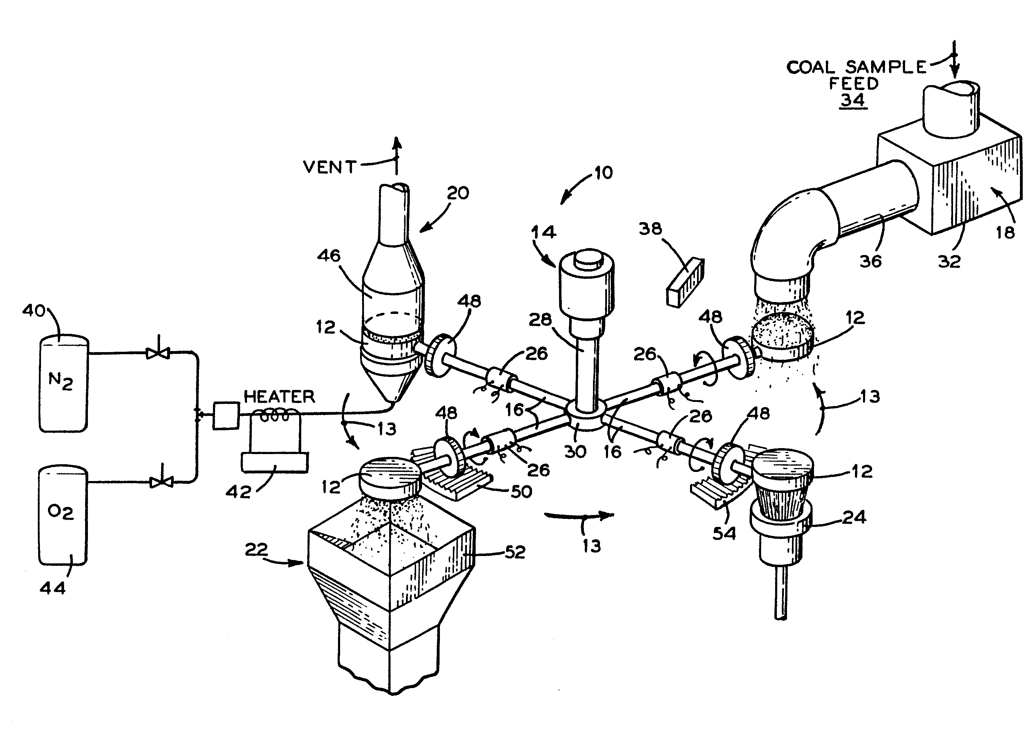

The invention will now be further described, by way of illustrative and non-limiting example, with referance to the accompanying drawing, the sole figure of which is a perspective view schematically representing a preferred coal analysis apparatus embodying the invention.

The drawing shows an on-line coal analysis apparatus or system 10 which comprises: a plurality of sampling cups 12, which are indexed along a circular path depicted by the arrows 13 by means of a central indexing motor drive unit 14 which drives the cups through radially extending arms or shafts 16; and a plurality of functional stations including a sampling station 18, an analysing station 20, a dumping station 22 where sample residue is dumped into a waste hopper, and a cleaning station 24 where each sample cup is cleaned for re-use in another analysing cycle.

Each of the sampling cups 12 is a shallow open cylinder made from a porous metal such as sintered stainless steel, inconel, or another durable, inert material. Each of the radial arms 16 has a strain gauge 26 or other suitable device attached to it and connected to a central controller through slip rings or the like to provide accurate monitoring and recording of the weight of each sample cup during the various steps of an analysing cycle.

The indexing motor drive unit 14 has a vertical output shaft 28 with a cylindrical head 30 attached to the lower end thereof. As illustrated herein, four radial arms 16 extend horizontally from the head 30, and a sampling cup 12 is fixed to the end of each arm. As will be described in further detail below, the arms 16 are mounted to be rotatable about their longitudinal axes within the head 30.

The sampling station 18 comprises a known type of pulveriser 32 for reducing the coal sample to a particle size suitable for chemical analysis, an inlet feed tube 34 leading from a main coal feed system of the facility, and an outlet tube 36 arranged to direct a pulverised coal sample to a waiting cup 12 which has been indexed into position beneath the outlet tube 36. The coal sample can be extracted from the coal feed system continuously by means of an auger or other such known device.

At sampling station the cup 12 is intentionally overfilled, the excess being returned to the main feed system. Once the cup 12 is filled the indexing unit 14 is energised to rotate the cup towards the analysing station 20. To ensure that a uniform sample is collected, a stationary scraper 38 is mounted between the sampling and analysing stations 18, 20 and is positioned to scrape off the excess sample level with the top of the cup 12 to obtain a known sample volume. Since the volume of the cup 12 is known and the weight of the sample can be determined by means of the strain gauge 26 on the arm 16, the bulk density of the sample can be calculated, for example by means of a microprocessor, in accordance with the equation:

$$\text{bulk density} = \frac{\text{total weight} - \text{tare weight}}{\text{volume}}$$

At the analysing station 20 the sample is first dried, which is done by passing nitrogen or other inert gas from a source 40 through a heater 42 and then through the porous sample cup 12. During the drying operation the weight is monitored and the drying operation is stopped when no further

4

significant weight loss is detected. The moisture content is then calculated by a microprocessor in accordance with the equation:

$$\text{Percent Moisture} = 100 \times \frac{\text{initial sample weight} - \text{final sample weight}}{\text{initial sample weight}}$$

It is understood that during the above drying process the temperature of the nitrogen is controlled to prevent chemical decomposition of the coal.

After drying, the sample is pyrolised by passing oxygen from a source 44 through the heater 42 and then through the sample.

Analyses of the carbon monoxide, carbon dioxide, sulphur dixoxide and nitrogen oxides are also performed at the analysing station 20. Oxygen content is analysed and used to control the incoming flow rate. Analyses of carbon monoxide, sulphur and nitrogen oxides can be performed using catalytic sensors placed in a vent hood 46 disposed over the sample at the analysing station 20. Oxygen is measured with a known zirconia-based sensor. Electro-chemical carbon dioxide sensors can also be employed.

Other known analytical methods can also be used, for example infrared spectroscopy for carbon monoxide and carbon dioxide, and ultraviolet and visible spectroscopy for sulphur and nitrogen oxides.

Constituent concentrations are totalised during pyrolysis. The process is complete when the sample weight has stabilised. Knowing the total volume of oxygen added, a material balance can be run and the total concentration of each constituent in the sample calculated. The remaining sample weight is the ash content of the sample.

Using the temperature rise information and the total volume of oxygen added, a heat balance can be run to yield the calorific content of the coal sample.

After the analyses are completed the indexing monitor drive unit 14 is energised to rotate the sample cup 12 to the dumping station 22. To permit the cup 12 to be inverted over the dumping station 22, a gear 48 is mounted on each arm 16 and a rack 50 is located adjacent the dumping station. As the arm approaches the station 22, the gear 48 contacts the rack 50 and rotates $180^{\circ}$ when the cup 12 reaches a waste hopper 52 to dump the residue.

The sample cup 12 is then indexed to the cleaning station 24 where mechanical, ultrasonic, and/or chemical means are used to clean the cup

5

until its tare weight returns to within a predetermined range. Adjacent the cleaning station 24 a second rack 54 is located to rotate the cup 12 to its normal, upright position for refilling at the sampling station 18 in preparation for a new analysis cycle.

In the illustrated embodiment all analytical steps are indicated as being performed at a single analysing station 20. However, it should be appreciated that additional analysing stations can be provided to analyse other constituents and properties of the coal sample.

6

## CLAIMS

1. A method for the on-line analysis of coal, the method being characterised by the steps of filling a sample cup (12) of known volume with pulverised coal at a first station (18), indexing the sample cup (12) to a second station (20), performing one or more analysis operations on the coal sample at the second station (20), indexing the sample cup (12) to a third station (22), dumping the residue of the coal sample at the third station (22), indexing the sample cup (12) to a fourth station (24), cleaning the sample cup at the fourth station (24), and returning the sample cup (12) to the first station (18).

2. A method according to claim 1, wherein the sample cup (12) is initially overfilled at the first station (18) and the sample is levelled to the top of the sample cup between the first and second stations (18, 20) to provide a known volume of coal for analysis.

3. A method according to claim 1 or claim 2, wherein the weight of the sample cup (12) is monitored continuously.

4. A method according to claim 3, wherein the cleaning step is carried out until the weight of the sample cup (12) is within a predetermined range of the original tare weight of the sample cup.

5. A method according to any one of the preceding claims, wherein a plurality of the sample cups (12) are indexed simultaneously whereby the filling, analysis, dumping and cleaning steps are performed substantially simultaneously.

6. Apparatus for the on-line analysis of coal, the apparatus (10) being characterised by a sample cup (12) of predetermined volume, a first station (18) including means for introducing a sample of pulverised coal into the sample cup (12), a second station (20) including means for performing one or more analysis operations on the sample, a third station (22) including means for receiving the residue of the sample after completion of the analysis operation or operations, a fourth station (24) including means for cleaning the sample cup (12), and means for indexing the sample cup (12) sequentially from the first through to the fourth stations and back to the first station (18).

7. Apparatus according to claim 6, wherein the first, second, third and fourth stations (18, 20, 22, 24) are arranged along a circular path (13), the indexing means comprises an indexing motor (14) disposed centrally of the circular path (13) and an arm (16) extending radially from an output shaft (28) of the indexing motor (14), and the sampling cup (12) is attached to the arm (16) in a position to traverse the circular path (13).

8. Apparatus according to claim 7, wherin the means for introducing pulverised coal into the sampling cup (12) is operable to overfill the sampling cup, the sampling cup (12) comprises a cylinder open at one end, and scraper means (38) is disposed along the circular path (13) in a position to scrape off the overfilled portion of the sample level with the open end of the sampling cup (12).

9. Apparatus according to claim 7 or claim 8, wherein the arm (16) is rotatable about its longitudinal axis and means is provided adjacent the third and fourth stations (22, 24) to engage the arm (16) to rotate the arm as the sampling cup (12) traverses the circular path (13).

10. Apparatus according to claim 9, wherein a gear (48) is fixed to the arm (16) for rotation therewith, and the means to rotate the arm comprises racks (50, 54) arranged in position along the circular path (13) to engage the gear (48).

11. Apparatus according to any one of claims 7 to 10, including means (26) on the arm (16) for providing a continuous indication of the weight of the sampling cup (12).

12. Apparatus according to any one of claims 7 to 11, including a plurality of arms (16) extending radially from the output shaft (28) of the indexing motor (14), each of the arms (16) having a sampling cup (12) fixed thereto and the arms being arranged to simultaneously position a sampling cup (12) at each of the stations (18, 20, 22, 24).

13. Apparatus according to any one of claims 6 to 12, wherein the sampling cup (12) is formed of a porous material, whereby one or more gases may be passed through the cup and into the sample therein.

COAL SAMPLE
FEED
34

VENT

N₂

O₂

HEATER

0099202

1/1

0099202

EUROPEAN SEARCH REPORT

Application number

EP 83 30 3724

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-1 379 146 (CIMENTS LAFARGE S.A.) * Claims 1-5; figure 1 * | 1,6 | G 01 N 33/22<br>G 01 N 1/20 |
| A | US-A-3 481 709 (T.J. SLONE) | | |
| A | DE-A-1 914 929 (SIEMENS AG) | | |
| A | US-A-4 055 259 (J. SIBRAVA) | | |

TECHNICAL FIELDS SEARCHED (Int. Cl. 3)

G 01 N 1/20
G 01 N 33/22

The present search report has been drawn up for all claims

| Place of search | Date of completion of the search | Examiner |
|---|---|---|
| BERLIN | 22-09-1983 | SCHWARTZ K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03 82